# EUROPEAN PATENT APPLICATION

(11) **EP 0 732 079 A1**
(43) Date of publication of application: **18.09.1996**
(21) Application number: 96200667.2
(22) Date of filing: 12.03.1996
(51) Int. Cl.: A61B 17/36, A61F 2/06

(54) **Balloon catheter with light conductor**

(30) Priority: 13.03.1995 NL 9500494
(71) Applicant: CORDIS EUROPA N.V., NL-9301 LJ Roden (NL)
(72) Inventor: Ligtenberg, Hendrikus Cornelis Geert, 9351 PX Leek (NL); Haan, Marcel Gerard, 9312 PE Nietap (NL)
(74) Representative: 't Jong, Bastiaan Jacobus

(57) **Abstract**

The invention relates to a catheter comprising a tube-like basic body with a distal and a proximal end and a balloon member arranged at the distal end. A light conductor extends from the proximal to the distal end which has close to the proximal end a light-absorbing end-section and close to the distal end a light-emitting end-section positioned inside the balloon member. The catheter comprises furthermore a light sensor, with a light-sensitive element situated inside the balloon member and a conductor conducting a light-dependent signal to the proximal end.

## Description

The invention relates to a balloon catheter comprising a tube-like basic body with a distal and a proximal end. At the distal end a balloon member has been arranged in the usual manner. A light conductor extends from the proximal end to the distal end, which has a light-absorbing end-section at the proximal end. Light absorbed by the light-absorbing end-section is conducted via the conductor to its light-emitting end-section, which is situated inside the balloon member. The catheter comprises furthermore a light sensor with a light-sensitive element received inside the balloon member, and a conductor conducting a light-dependent signal to the proximal end.

Such a catheter is known from US-A-5 125 925.

With a catheter of this kind it is possible to fit for instance a stent, made of a plastic material which cures under the action of light, in particular material which cures under the action of UV-light, inside the body of a patient following expansion. The balloon member can be used to effect the expansion. As a result of the light conducted via the light conductor the material of which the stent has been made cures, so that it retains its expanded form.

During the treatment information can be obtained by means of the light sensor as to the intensity of the quantity of light actually emitted by the light-emitting end of the light conductor, so that the intensity and quantity of the light emitted can be controlled.

With the catheter according to the invention as characterized in claim 1 a good relation is achieved between the light-emitting end-section of the light conductor and the light-absorbing end-section of the sensor, so that a high degree of accuracy of the light values measured can be realised.

With the measure as set out in claim 2 it is achieved that the sensor can measure the intensity of the quantity of residual light emerging from the end-face of the light conductor, so that it is possible to accurately determine the quantity of light actually emitted. This equals the light energy supplied minus the light energy removed by the sensor.

A very suitable embodiment is characterised in claim 3. No measuring errors will occur due to unknown emission of light at the transition between the two conductors, which will add to the accuracy of the measurement.

The measure as set out in claim 4 is preferably employed. Because the light-emitting end-section emits the light in a radial direction, the element to be exposed, in particular the stent, will be exposed evenly, so that the curing will take place uniformly.

The invention will be explained in greater detail in the following description with reference to the attached drawings.
- Figure 1: illustrates partly schematically a catheter according to the invention, whereby the distal end-section is illustrated at an enlarged scale.
- Figure 2: shows a view corresponding to figure 1 of an end-section of an alternative embodiment.
- Figure 3: shows a view corresponding to figure 1 of an end-section of yet another embodiment.

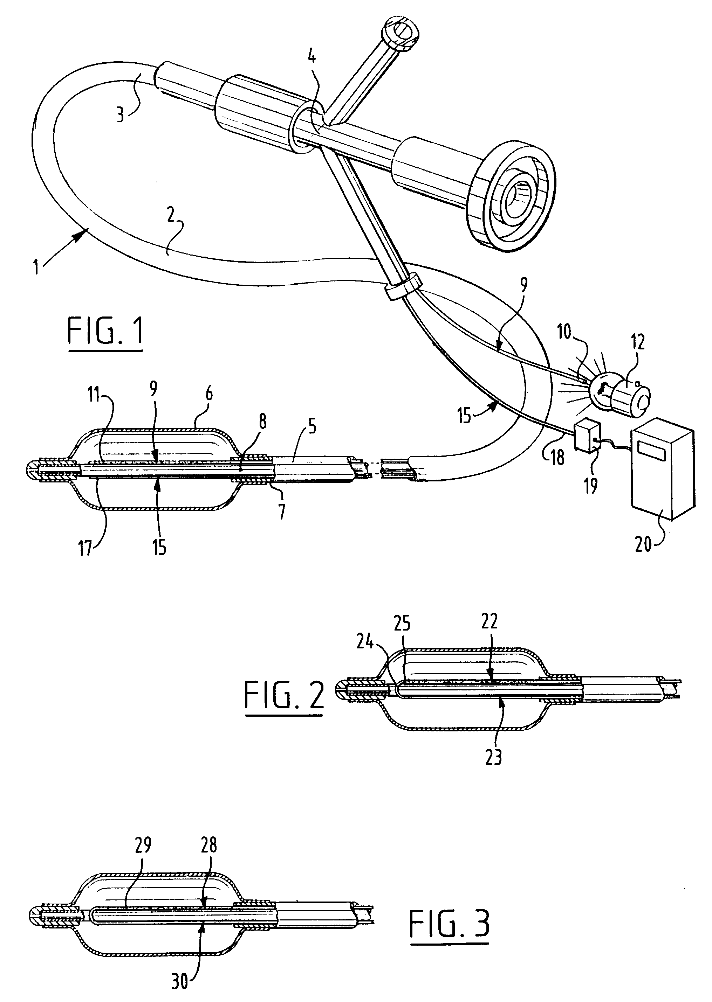

The catheter 1 according to the invention as illustrated in figure 1 comprises in the usual manner a tube-like basic body 2 with a proximal end 3 and a distal end 5. The proximal end 3, which remains outside the body of a patient during treatment, is provided with a connecting member 4 for one or more lumens inside the basic body.

At the distal end 5 the basic body supports a balloon member 6. As can be seen in figure 1, the basic body 2 is made up of an outer tube-like member 7, inside a lumen whereof an inner tube-like member 8 has been received. The balloon member 6 has been arranged with its relatively proximal end to the end of the outer tube-like member 7 and is, with its relatively distal end, connected to the distal end of the inner tube-like member 8. Via the channel remaining inside the lumen of the outer tube-like member 7, a liquid or gas under pressure can be supplied from the connecting member 4 to the balloon 6, in order to expand the latter. The inner tube-like member also comprises a lumen, which can serve as guiding channel for a guide wire.

The catheter 1 comprises furthermore a first light conductor 9 which extends from the proximal end of the catheter to the distal end 5 thereof. At the proximal end 3 the light conductor 9 is led outside through the connecting member 4. The proximal end of the light conductor 9 forms a light absorbing end-section 10, which has been made in such a way that it can conduct light, emitted by a source of light 12 illustrated schematically here, to the light conductor 9.

At the opposite, distal end-section, the light conductor 9 comprises a light-emitting end 11, which is situated inside the balloon member 6. This light-emitting end 11 has been treated in such a manner, in particular, roughened by grinding, that the light conducted through the conductor is emitted from the light-emitting end-section 11 in a radial direction.

The catheter 1 is furthermore provided with a light sensor, with which the quantity of light emitted by the light-emitting end-section 11, and in particular the intensity thereof, can be determined. This sensor comprises a second light conductor 15, comprising a light-absorbing end 17, also situated inside the balloon member 6, and at the opposite, proximal end a light-emitting end-section 18. In this embodiment a light-sensitive cell 19 is connected to the light-emitting end-section 18. This light-sensitive cell 19 is in turn connected to a processing device 20, which processes the signal coming from the light-sensitive cell 19.

Thus the second light conductor 15 forms a light sensor of which the light-absorbing end 17 is a light-sensitive element; the section of the conductor extending through the basic body 2 to the proximal end forms a conductor conducting a light-dependent signal to this proximal end.

With another embodiment not shown here, the light sensor can also comprise a light-sensitive cell inside the balloon member 6, which conducts the light-dependent signal via electrical conductors to the proximal end, for further processing.

The processing device 20 gives an indication of, in particular, the intensity of the light inside the balloon member 6. Depending on the value measured, the light source 12 can be controlled in order to obtain the right intensity required for the purpose of curing the plastic material of which the stent has been made. It is obviously possible to connect the processing device 20 directly to the light source 12, so that a feedback control system for the light intensity is obtained.

With the embodiment shown in figure 1, the light absorbing end 17 of the second light conductor 15 absorbs light along at least a significant section of the end extending inside the balloon member 6. The value measured consequently depends on the actual light intensity inside the balloon member.

It is however also possible to detect the quantity of light not emitted by a light conductor, in order to deduce from that and from the light intensity supplied, how much light has actually been emitted.

This has been employed with the embodiments illustrated in the figures 2 and 3.

In figure 2, the light conductor comprising the light-emitting end-section has been indicated with the number 22, and the second light conductor comprising the light-absorbing end-section with the number 23. The end-face of the second light conductor 23 is connected to the end-surface 25 of the first light conductor 22. The second light conductor 23 has been positioned, as illustrated, in a loop 24, in order to obtain a connection of which the constituent parts are positioned in line with each other.

With this embodiment it is achieved that the light sensor determines the quantity of residual light at the end surface of the first light conductor 22 supplying the light. Thus it can be determined how much light has been emitted by the light-emitting end of the light conductor 22.

As far as the principle involved is concerned, figure 3 shows an embodiment corresponding to the one of figure 2. In this case the light conductors form however one continuous light conductor 29 tracing a loop inside the balloon member. The first light conductor 28 forms the supplying light conductor and the section 30, extending in a parallel fashion, forms the second, light-removing light conductor.

The light-emitting end-sections of the first light conductor have been treated every time in such a way that the light conveyed by the light conductor is emitted radially in this light-emitting end-section. This can be achieved for instance by roughening the surface of the light conductor by for instance grinding.

## Claims

1. Catheter comprising a tube-like basic body with a distal and a proximal end and a balloon member arranged at the distal end, wherein a light conductor extends from the proximal to the distal end and has close to the proximal end a light-absorbing end-section and close to the distal end a light-emitting end-section positioned inside the balloon member and furthermore comprising a light sensor, with a light-sensitive element situated inside the balloon member and a conductor conducting a light-dependent signal to the proximal end, said light sensor comprising a second light conductor, extending from the distal to the proximal end and has a light-absorbing end-section close to the distal end and a light-emitting end-section close to the proximal end, characterized in that the light-absorbing end-section of the second light conductor makes contact with the light-emitting end-section of the first light conductor.

2. Catheter as claimed in claim 1, wherein the second light conductor is connected end-surface to end-surface with the first light conductor.

3. Catheter as claimed in claim 2, wherein the first and the second light conductor form one continuous light conductor, which traces a loop inside the balloon member.

4. Catheter as claimed in one of the previous claims, wherein the light-emitting end-section of the first light conductor has been made in such a way that it can emit light in a radial direction.

5. Catheter as claimed in one of the previous claims, wherein at least the first light conductor comprises an optic fibre bundle and the surface of its light-emitting end-section has been roughened.
